# EUROPEAN PATENT SPECIFICATION

(11) **EP 3 380 237 B1**
(45) Date of publication and mention of the grant of the patent: **02.09.2020**
(21) Application number: 16804862.7
(22) Date of filing: 23.11.2016
(51) Int. Cl.: B01L 3/00, C12Q 1/37, G01N 33/558

(54) **METHOD OF DETECTING PERITONITIS**
VERFAHREN ZUR ERKENNUNG VON PERITONITIS
MÉTHODE DE DÉTECTION DE LA PÉRITONITE

(30) Priority: 23.11.2015 GB 201520657
(43) Date of publication of application: 03.10.2018
(73) Proprietor: Mologic Ltd, Thurleigh, Bedfordshire MK44 2YP (GB)
(72) Inventor: PAREKH, Gita, Thurleigh, Bedfordshire MK44 2YP (GB)
(74) Representative: Boult Wade Tennant LLP
(86) International application number: PCT/GB2016/053684
(87) International publication number: WO 2017/089801

(56) References cited:
- EP-A1- 2 676 606
- WO-A1-95/29756
- WO-A1-2015/017591
- AU-A1- 2004 203 639
- JP-A- 2001 066 306
- JP-A- 2001 066 306
- US-A1- 2002 173 047
- SHI-KUN YANG ET AL: "Significance of serum procalcitonin as biomarker for detection of bacterial peritonitis: a systematic review and meta-analysis", BMC INFECTIOUS DISEASES, BIOMED CENTRAL, LONDON, GB, vol. 14, no. 1, 22 August 2014 (2014-08-22), page 452, XP021195620, ISSN: 1471-2334, DOI: 10.1186/1471-2334-14-452
- YUTAKA TAKEZAWA ET AL: "Matrix metalloproteinase levels in peritoneal effluents were increased in a patient with appendicitis undergoing continuous ambulatory peritoneal dialysis", CLINICAL AND EXPERIMENTAL NEPHROLOGY ; OFFICIAL PUBLICATION OF THE JAPAN SOCIETY OF NEPHROLOGY, SPRINGER-VERLAG, TO, vol. 16, no. 3, 21 December 2011 (2011-12-21), pages 501-504, XP035073275, ISSN: 1437-7799, DOI: 10.1007/S10157-011-0576-0
- ANNELI LAUHIO ET AL: "Serum MMP-8, -9 and TIMP-1 in sepsis: High serum levels of MMP-8 and TIMP-1 are associated with fatal outcome in a multicentre, prospective cohort study. Hypothetical impact of tetracyclines", PHARMACOLOGICAL RESEARCH, vol. 64, no. 6, 2011, pages 590-594, XP028314229, ISSN: 1043-6618, DOI: 10.1016/J.PHRS.2011.06.019 [retrieved on 2011-07-01]
- ELINE DEJONCKHEERE ET AL: "Matrix metalloproteinase8 has a central role in inflammatory disorders and cancer progression", CYTOKINE AND GROWTH FACTOR REVIEWS, vol. 22, no. 2, 2011, pages 73-81, XP028223934, ISSN: 1359-6101, DOI: 10.1016/J.CYTOGFR.2011.02.002 [retrieved on 2011-02-16]
- S.O. NWHATOR ET AL: "Clinical Correlates of a Lateral-Flow Immunoassay Oral Risk Indicator", JOURNAL OF PERIODONTOLOGY., vol. 85, no. 1, 1 January 2014 (2014-01-01), pages 188-194, XP55600926, US ISSN: 0022-3492, DOI: 10.1902/jop.2013.130116
- MENG-RUI LEE ET AL: "Plasma Biomarkers Can Predict Treatment Response in Tuberculosis Patients : A Prospective Observational Study", MEDICINE., vol. 94, no. 39, 1 September 2015 (2015-09-01), page e1628, XP55600964, US ISSN: 0025-7974, DOI: 10.1097/MD.0000000000001628
- JINGJING ZHANG ET AL: "Machine-learning algorithms define pathogen-specific local immune fingerprints in peritoneal dialysis patients with bacterial infections", KIDNEY INTERNATIONAL, vol. 92, no. 1, 1 July 2017 (2017-07-01), pages 179-191, XP55600968, LONDON, GB ISSN: 0085-2538, DOI: 10.1016/j.kint.2017.01.017
- SHI-KUN YANG ET AL: "Significance of serum procalcitonin as biomarker for detection of bacterial peritonitis: a systematic review and meta-analysis", BMC INFECTIOUS DISEASES, BIOMED CENTRAL, LONDON, GB, vol. 14, no. 1, 22 August 2014 (2014-08-22), page 452, XP021195620, ISSN: 1471-2334, DOI: 10.1186/1471-2334-14-452
- YUTAKA TAKEZAWA ET AL: "Matrix metalloproteinase levels in peritoneal effluents were increased in a patient with appendicitis undergoing continuous ambulatory peritoneal dialysis", CLINICAL AND EXPERIMENTAL NEPHROLOGY ; OFFICIAL PUBLICATION OF THE JAPAN SOCIETY OF NEPHROLOGY, SPRINGER-VERLAG, TO, vol. 16, no. 3, 21 December 2011 (2011-12-21), pages 501-504, XP035073275, ISSN: 1437-7799, DOI: 10.1007/S10157-011-0576-0
- ANNELI LAUHIO ET AL: "Serum MMP-8, -9 and TIMP-1 in sepsis: High serum levels of MMP-8 and TIMP-1 are associated with fatal outcome in a multicentre, prospective cohort study. Hypothetical impact of tetracyclines", PHARMACOLOGICAL RESEARCH, vol. 64, no. 6, 2011, pages 590-594, XP028314229, ISSN: 1043-6618, DOI: 10.1016/J.PHRS.2011.06.019 [retrieved on 2011-07-01]
- ELINE DEJONCKHEERE ET AL: "Matrix metalloproteinase8 has a central role in inflammatory disorders and cancer progression", CYTOKINE AND GROWTH FACTOR REVIEWS, vol. 22, no. 2, 2011, pages 73-81, XP028223934, ISSN: 1359-6101, DOI: 10.1016/J.CYTOGFR.2011.02.002 [retrieved on 2011-02-16]
- S.O. NWHATOR ET AL: "Clinical Correlates of a Lateral-Flow Immunoassay Oral Risk Indicator", JOURNAL OF PERIODONTOLOGY., vol. 85, no. 1, 1 January 2014 (2014-01-01), pages 188-194, XP055600926, US ISSN: 0022-3492, DOI: 10.1902/jop.2013.130116
- MENG-RUI LEE ET AL: "Plasma Biomarkers Can Predict Treatment Response in Tuberculosis Patients : A Prospective Observational Study", MEDICINE., vol. 94, no. 39, 1 September 2015 (2015-09-01), page e1628, XP055600964, US ISSN: 0025-7974, DOI: 10.1097/MD.0000000000001628
- JINGJING ZHANG ET AL: "Machine-learning algorithms define pathogen-specific local immune fingerprints in peritoneal dialysis patients with bacterial infections", KIDNEY INTERNATIONAL, vol. 92, no. 1, 1 July 2017 (2017-07-01), pages 179-191, XP055600968, LONDON, GB ISSN: 0085-2538, DOI: 10.1016/j.kint.2017.01.017

## Description

### FIELD OF THE INVENTION

The present invention relates to the testing of peritoneal dialysate for peritonitis.

### BACKGROUND

Peritoneal Dialysis (PD) is the preferred management method for kidney failure patients in the home environment, enabling patients to maintain some semblance of a normal life and regular employment. PD is a passive activity, requiring multiple (typically 4) bag changes a day to remove toxins from the blood stream. PD is an alternative to haemodialysis and involves the use of the patient's peritoneum in the abdomen as an exchange membrane for exchanging substances with the blood. A catheter is surgically implanted in the patient permitting fluid access to the peritoneal cavity. Fluid is then flushed into and out of the abdomen either at night during sleep, known as Automated Peritoneal Dialysis, or continuously, known as Continuous Ambulatory Peritoneal Dialysis.

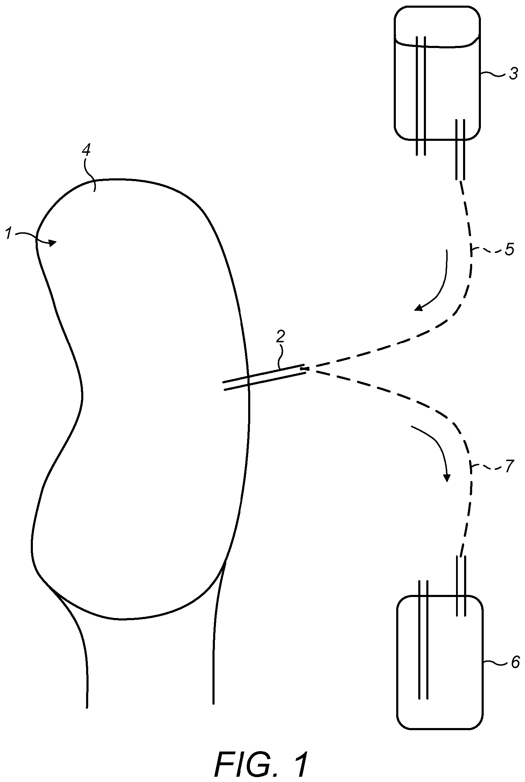

The PD procedure is shown schematically in Figure 1, which illustrates a patient 1, catheter 2, dialysis fluid bag 3 containing the fluid to be passed into the abdominal cavity 4 via feed line 5 and the dialysate fluid bag 6 containing the waste fluid withdrawn from the abdominal cavity 4 via a drain line 7. PD involves relatively large volumes of fluid. Each exchange of fluid into and out of the abdomen may by up to 2.5 litres or more. Thus the dialysate bag 6 may be of a large capacity - typically 0.5 to 2.5 litres or more. For convenience and hygiene the fluid drained from the abdominal cavity is passed directly to the dialysate bag 6 via the drain line 7.

Unfortunately, infection is the primary cause for patients to lose the freedom of PD, as it causes damage to the peritoneum and a loss of the infusion line/catheter. Costs of treatment are high and, where the line is lost, surgical intervention is required.

Shi-Kun Yang et al, "Significance of serum procalcitonin as biomarker for detection of bacterial peritonitis- a systematic review and meta-analysis" BMC Infectious Diseases, Biomed Central, GB, vol 14, 1, p452 discloses procalcitonin as a biomarker for bacterial peritonitis.

Yutaka Takezawa et al. Clinical and Experimental nephrology vol 16(3) (2011) p 501-504 discloses that matrix metalloproteinase levels in peritoneal effluents were increased in a patient with appendicitis undergoing continuous ambulatory peritoneal dialysis.

Anneli Lauhio et al. Pharmacological Research vol 64, no. 6, pages 590-594 discloses that high serum levels of MMP-8 and TIMP_1 are associated with fatal outcome in certain sepsis patients.

### SUMMARY OF THE INVENTION

Catching an infection early would prevent damage and line loss yet there is no way for a patient to detect an emergent infection other than a change in fluid colour or aroma at discharge. The methods of the present invention can integrate with PD fluid-handling equipment, in particular the bags and associated tubing. In some embodiments, the methods interface with the waste tube of a PD fluid bag and test the waste fluid as it is discarded. The user will be able to observe a simple visual result and take corrective action long before an infection takes hold. The patient will be able to remain at home, reducing healthcare costs and extending the clinical utility of PD.

The methods provide reliable detection of the first measurable response of the innate immune system, namely neutrophil infiltration.

The invention provides a method of detecting peritonitis caused by an infection in a subject suffering from kidney disease and undergoing peritoneal dialysis, the method comprising determining the level of at least the marker MMP8 in a sample of peritoneal dialysate wherein an increased level of at least MMP8 is indicative of peritonitis caused by an infection.

The method may further comprise selecting the subject for treatment with an antibiotic based on an increased level of at least MMP8 in the sample of peritoneal dialysate.

The method may comprise determining the level of at least two markers in a sample of peritoneal dialysate, wherein one of the markers is MMP8 and the other marker is selected from IL-6, HNE, MMP2, MMP9, TIMP1, TIMP2, NGAL, A1AT, desmosine, fibrinogen, IL-8, calprotectin, fMLP, IL1b, cystatin C, HSA, RBP4, SPD, MPO, sICAM and TNFa, wherein an increased level of MMP8 and at least one of IL-6, HNE, MMP2, MMP9, TIMP1, TIMP2, NGAL, A1AT, desmosine, IL-8, calprotectin, fMLP, IL1b, cystatin C, HSA, RBP4, SPD, MPO, sICAM and TNFa and/or a decreased level of fibrinogen is indicative of peritonitis caused by an infection.

Preferably, the at least one marker is selected from MMP8 and the other marker is selected from HNE, MMP2, MMP9, IL-6, calprotectin and MPO.

Preferably, the level of the markers MMP8 and IL-6 is determined in a sample of peritoneal dialysate.

The method may comprise detecting peritonitis at an early stage.

The method may be performed in a lateral flow assay format.

The method may be performed on a bulk source of fluid using a diagnostic test apparatus of the type comprising:
an elongate housing defining a test strip holder containing a lateral flow test strip, a fluid sampling chamber provided with an opening connecting the fluid sampling chamber with the test strip holder, and a viewing window in the elongate housing allowing reading of one or more portions of the lateral flow test strip; the method comprising the steps of:
coupling the diagnostic test apparatus to the bulk source of fluid by use of a connector;
during or after coupling of the connector, establishing a fluid communication between the bulk source of fluid and the fluid sampling chamber of the diagnostic test apparatus to convey fluid from the bulk source of fluid into the fluid sampling chamber;
causing fluid to pass from the fluid sampling chamber through the opening to wet the lateral flow test strip.

Preferably, said method may be performed using a diagnostic test apparatus comprising:
an elongate housing defining a test strip holder containing a lateral flow test strip;
a fluid sampling chamber provided with an opening connecting the fluid sampling chamber with the test strip holder;
a viewing window in the elongate housing allowing reading of one or more portions of the lateral flow test strip; and
a connector configured to couple the diagnostic test apparatus to a bulk source of fluid.

The present invention also provides an antibiotic for use in a method of treating peritonitis, wherein the subject suffers from kidney disease which is managed by peritoneal dialysis and has been selected for treatment by performing the method of the invention as described above.

Preferably, the subject displays an increased level of at least MMP8 and IL-6.

Preferably, the antibiotic is:
a) selected from an aminoglycoside, a cephalosporin, a glycopeptide, a penicillin, a quinolone, aztreonam, clindamycin, imipenem-cilastin, linezolid, metronidazole, rifampin and an antifungal and/or
b) administered intraperitoneally, orally or intravenously (preferably intraperitoneally); and/or
c) administered during peritoneal dialysis and/or
d) administered with the dialysis fluid utilised in the peritoneal dialysis and/or
e) administered separately from the dialysis fluid utilised in the peritoneal dialysis.

According to the invention, the sample is a peritoneal dialysate. Thus, the methods are performed as in vitro methods using the isolated sample, which can for example be retrieved from a dialysate fluid bag.

Peritonitis is defined as inflammation of the peritoneum and the methods of the invention are concerned with peritonitis caused by an infection. The subject is suffering from kidney disease. In some embodiments, the kidney disease is chronic and/or severe.

Thus, the inveniton is concerned with the detection of infections, a common problem with peritoneal dialysis. Subjects found to be suffering from peritonitis may need to be treated, for example with an appropriate antibiotic.

Any suitable antibiotic may be employed to treat peritonitis as discussed for example in Warady et al (2012) Peritoneal Dialysis International, Vol. 32, pp. S32-S86 doi: 10.3747/pdi.2011.00091. Thus, in some embodiments of the invention, the antibiotic is selected from an aminoglycoside, a cephalosporin, a glycopeptide, a penicillin, a quinolone, aztreonam, clindamycin, imipenem-cilastin, linezolid, metronidazole, rifampin and an antifungal. Combinations are also envisaged within the scope of the invention.

In certain embodiments, the aminoglycoside is selected from gentamicin, netilmycin, tobramycin and amikacin. In some embodiments, the cephalosporin is selected from cefazolin, cefepimine, cefotaxime and ceftazidimine. In some embodiments, the glycopeptide is selected from vancomycin and teicoplanin. In some embodiments, the antifungal is selected from fluconazole and caspofungin.

Any suitable route of administration may be employed. In some embodiments, the antibiotic is to be administered intraperitoneally, orally or intravenously. Intraperitoneal administration may be most convenient for PD patients. In some embodiments, the antibiotic may be administered with the dialysis fluid. However, some antibiotics such as aminoglycosides and penicillins should not be mixed in dialysis fluid because of the potential for inactivation.

Suitable dosing regimens can be readily derived by one of skill in the art taking into account dosing instructions and subject characteristics. Some dosing recommendations useful in the present invention are shown in Table 1 below, derived from Warady et al:

**Antibiotic Dosing Recommendations^{a} for the Treatment of Peritonitis**

| | | | |
|---|---|---|---|
| | Therapy type | | |
| | Continuous^{b} | | |
| Antibiotic type | Loading dose | Maintenance dose | Intermittent^{b} |

| Aminoglycosides (IP)^{c} | | | |
|---|---|---|---|
| Gentamicin | 8 mg/L | 4 mg/L | |
| Netilmycin | 8 mg/L | 4 mg/L | Anuric: 0.6 mg/kg |
| Tobramycin | 8 mg/L | 4 mg/L | Non-anuric: 0.75 mg/kg |
| Amikacin | 25 mg/L | 12 mg/L | |
| | | | |

| Cephalosporins (IP) | | | |
|---|---|---|---|
| Cefazolin | 500 mg/L | 125 mg/L | 20 mg/kg |
| Cefepime | 500 mg/L | 125 mg/L | 15 mg/kg |
| Cefotaxime | 500 mg/L | 250 mg/L | 30 mg/kg |
| Ceftazidime | 500 mg/L | 125 mg/L | 20 mg/kg |
| | | | |

| GLycopeptides (IP)^{d} | | | |
|---|---|---|---|
| Vancomycin | 1000 mg/L | 25 mg/L | 30 mg/kg; repeat dosing: 15 mg/kg every 3-5 days |
| Teicoplanin^{e} | 400 mg/L | 20 mg/L | 15 mg/kg every 5-7 days |

| Penicillins (IP)^{c} | | | |
|---|---|---|---|
| Ampicillin | - | 125 mg/L | - |

| Quinolones (IP) | | | |
|---|---|---|---|
| Ciprofloxacin | 50 mg/L | 25 mg/L | - |
| | | | |

| Others | | | |
|---|---|---|---|
| Aztreonam (IP) | 1000 mg/L | 250 mg/L | - |
| Clindamycin (IP) | 300 mg/L | 150 mg/L | - |
| Imipenem-cilastin (IP) | 250 mg/L | 50 mg/L | - |
| Linezolid (P0) | <5 Years: 30 mg/kg daily, divided into 3 doses | | |
| | 5-11 Years: 20 mg/kg daily, divided into 2 doses | | |
| | ≥12 Years: 600 mg/dose, twice daily | | |
| Metronidazole (P0) | 30 mg/kg daiLy, divided into 3 doses (maximum: 1.2 g daily) | | |
| Rifampin (P0) | 10-20 mg/kg daiLy, divided into 2 doses (maximum: 600 mg daiLy | | |
| | | | |

| Antifungals | | | |
|---|---|---|---|
| Fluconazole (IP, IV, or P0) | 6-12 mg/kg every 24-48 h (maximum: 400 mg daily) | | |
| Caspofungin (IV only) | 70 mg/m² on day 1 (maximum: 70 mg daily) | 50 mg/m² daiLy (maximum: 50 mg daily) | |

| | | | |
|---|---|---|---|
| IP=intraperitoneally, IV= intravenously, P0=orally. ^{a} Adapted from Li et al. (7), The Renal Drug Reference Guide (171), and Taketomo *et al*. (172). ^{b} For continuous therapy, the exchange with the loading dose shouLd dwell for 3-6 hours; all subsequent exchanges during the treatment course shouLd contain the maintenance dose. For intermittent therapy, the dose should be a pplied once daily in the long-dwell, unless otherwise specified. ^{c} Aminoglycosides and penicillins should not be mixed in dialysis fluid because of the potential for inactivation. ^{d} In patients with residual renal function, glycopeptide elimination may be accelerated. If intermittent therapy is used in such a setting, the second dose shouLd be time-based on a blood level obtained 2-4 days after the initial dose. Re-dosing should occur when the bLood level is <15 mg/L for vancomycin, or <8 m g/L for teicoplanin. Intermittent therapy is not recommended for patients with residual renal function unless serum levels of the drug can be monitored in a timely manner. ^{e} Teicoplanin is not currently available in the United States., | | | |

While each of the markers described herein have been shown to give useful information in relation to peritonitis detection, certain markers give highly significant results (P value <0.0001). As described herein, the most specific and sensitive marker determined by the inventors is MMP8. Accordingly, in all embodiments, the at least one marker is MMP8.

According to some embodiments of the invention, at least two markers are determined, provided at least one marker is MMP8. The two markers may be MMP-8 and IL-6 on one embodiment.

Increased and decreased levels of the markers are specified in relation to the corresponding peritoneal dialysate from a subject that is not suffering from peritonitis. As is shown herein, significantly increased (or decreased in the case of fibrinogen) levels of the markers can be used to sensitively and specifically identify infection at an early stage. This permits early and effective intervention and reduces unnecessary use of antibiotics where no infection is present. Cut off values for increased markers may be at the level of at least 0.1, 0.2, 0.3, 0.4, 0.5, 0.6, 0.7, 0.8, 0.9, 1, 2, 3, 4, 5, 6, 7, 8, 9, 10, 15, 20, 25, 50 or 100 ng/ml or more depending upon the marker concerned. The skilled person would be well able to determine an appropriate cut-off value given a known set of samples containing infections and control samples. Cut-off values may be adjusted to maximise sensitivity and/or specificity as required. For example, decision tree analysis may be used to determine a suitable cut-off. Background levels of some markers may be sufficiently low that any visible line on the test strip is indicative of peritonitis in some embodiments. Thus, in some embodiments, simple detection of the marker indicates peritonitis (and this is included within the definition of "increased" because the levels are higher than control). Alternatively, the various components of the test line may be included at concentrations such that threshold levels of the marker must be exceeded in order to give a visible test line. In specific embodiments, the threshold for MMP8 is around 0.4, such as 0.418 ng/ml.

The level of the markers may be determined at the level of protein or RNA (specifically mRNA). "Level" is also defined to include determining enzymatic activity of the relevant marker, as appropriate. This in some embodiments, the level of at least one marker is determined by an enzymatic activity assay. In some embodiments, zymography is utilised. This technique measures a number of hydrolytic enzymes, but in some embodiments measures MMP2 and/or MMP9 activity. In some embodiments a MMP substrate assay is utilised. Examples of suitable assays can be found in WO2009/024805, WO2009/063208, WO2007/128980, WO2007/096637, WO2013/156794, WO2013/156795 and WO2015/059487.

In some embodiments, the level of at least one marker is determined by an immunoassay. Many suitable immunoassay formats are known in the art, including ELISAs which may be sandwich ELISAs and/or competitive ELISAs.

In specific embodiments, the method is performed in a lateral flow assay format. Generally, therefore, the disclosure relies upon some form of solid support. The solid support may define a liquid flow path for the sample. In specific embodiments, the solid support comprises a chromatographic medium or a capillary flow device. The disclosure may be provided in a test strip format in some embodiments. As discussed herein, the lateral flow test strip incorporates a sample receiving zone (i.e. a region of the strip, towards the upstream end to which the sample is applied). The sample may be directly applied to the sample receiving zone. Alternatively, the sample may be transferred from the fluid sampling chamber of the device to the lateral flow test strip, for example via a wick element.

Downstream of the sample receiving zone is a test line. The test line shows a visible line in the presence of (increased levels of) the at least one marker in the sample. Threshold levels of the marker or markers corresponding to a visible test line may be readily determined by one skilled in the art as discussed above.

Multiple markers may be detected in a single test strip according to the invention. This may involve the use of multiple test lines as defined herein, one for each marker. Thus, the test strip may include a test line for at least two of the markers described herein. One of the markers is MMP-8. One of the markers may be IL-6. The test strip may include a test line for MMP-8 and IL-6 in preferred embodiments.

This test line comprises immobilized capture molecules. The capture molecules may be pre-immobilized at the test line or may become immobilized as liquid flows through the test strip. In the latter case, immobilization typically involves a specific binding interaction for example between a biotin molecule on the capture molecule and an avidin/streptavidin molecule immobilised in the test line. The capture molecules specifically bind to the marker or markers of interest where protein levels are to be determined. Where enzymatic activity is to be measured, the capture molecules may bind to a substrate or processed substrate molecule. Typically the capture molecules are antibodies or fragments or derivatives thereof.

The captured markers are then detected via a second specific binding interaction with a reporter molecule. The reporter molecule is also typically an antibody or fragment or derivative thereof. The reporter molecule is typically contained in the test strip upstream of the test sample. Upon hydration by the sample, the reporter molecule is carried along the test strip and interacts with the marker if present in the sample. It then becomes immobilised at the test line via the interaction between the capture molecule and the marker. Thus, typically, the immunoassay is a sandwich immunoassay. The reporter molecule may be labelled, either directly or indirectly. Suitable labels include gold particles and fluorophores. Indirect labels may be attached to a further binding molecule.

The test strip may also contain a control line downstream of the test line. Typically, this control line is used to confirm that the test has worked properly (i.e. that the sample has run through the entire test strip, past the test line). The control line may contain immobilized capture molecules in a similar fashion to those used at the test line (i.e. either pre-immobilized or which become immobilised during liquid flow through the device). The control line capture molecules may specifically bind to the reporter molecules. Thus, the reporter molecules that are not captured at the test line flow through and are captured at the test line. Alternatively, a separate control molecule may be included in the test strip. Upon hydration by the sample, the separate control molecule is carried along the test strip and specifically binds to the control line capture molecules. It then becomes immobilised at the control as a consequence. Again, the control molecules may be directly or indirectly labelled.

The test strip may also contain a sump downstream of the control line to absorb excess sample. The methods of the disclosure may be performed using large volumes of sample, because the peritoneal dialysate is of relatively large volume (of the order at least 100, 200, 300, 500, 1000, 2500 ml or more).

Suitable antibiotics are specified herein together with appropriate modes of administration and dosages. That discussion applies *mutatis mutandis* to these aspects of the disclosure. Thus, in some embodiments, the antibiotic is selected from an aminoglycoside, a cephalosporin, a glycopeptide, a penicillin, a quinolone, aztreonam, clindamycin, imipenem-cilastin, linezolid, metronidazole, rifampin and an antifungal. In some embodiments, the antibiotic is administered intraperitoneally, orally or intravenously (preferably intraperitoneally). In some embodiments, the antibiotic is administered during peritoneal dialysis. In certain embodiments, the antibiotic is administered with the dialysis fluid utilised in the peritoneal dialysis. In other embodiments, the antibiotic, optionally an aminoglycoside or penicllin, is administered separately from the dialysis fluid utilised in the peritoneal dialysis.

The methods are intended to be integrated with the diagnostic test apparatus and corresponding methods and kit of parts. In particular, the lateral flow test strip of the diagnostic test apparatus, kit of parts and methods may comprise the test strips hereinbefore described. Thus the discussion of those aspects represent preferred embodiments.

### Brief Description of the Drawings

The present disclosure will now be described, by way of example only, with reference to the accompanying drawings in which:
Figure 1: A schematic representation of peritoneal dialysis;
Figures 2 to 5: A first example of diagnostic test apparatus according to the present disclosure;
Figures 6 to 9: A second example of diagnostic test apparatus according to the present disclosure;
Figures 10 to 13: A third example of diagnostic test apparatus according to the present disclosure;
Figures 14 to 19: A fourth example of diagnostic test apparatus according to the present disclosure;
Figure 20A: Scatter graph showing median and interquartile range (IQR) generated from ELISA data comparing peritonitis vs stable individuals (Mann-Whitney test p<0.0001);
Figure 20B: Receiver Operator Characteristic (ROC) curves for the diagnosis of infection, area under the curve (AUC) values are shown;
Figure 21A: Scatter graph showing median and IQR generated from LF data comparing peritonitis vs stable individuals (Mann-Whitney test p<0.0001);
Figure 21B: ROC curve for the diagnosis of infection;
Figure 22: Decision tree analysis providing initial cut-off for levels of MMP8;

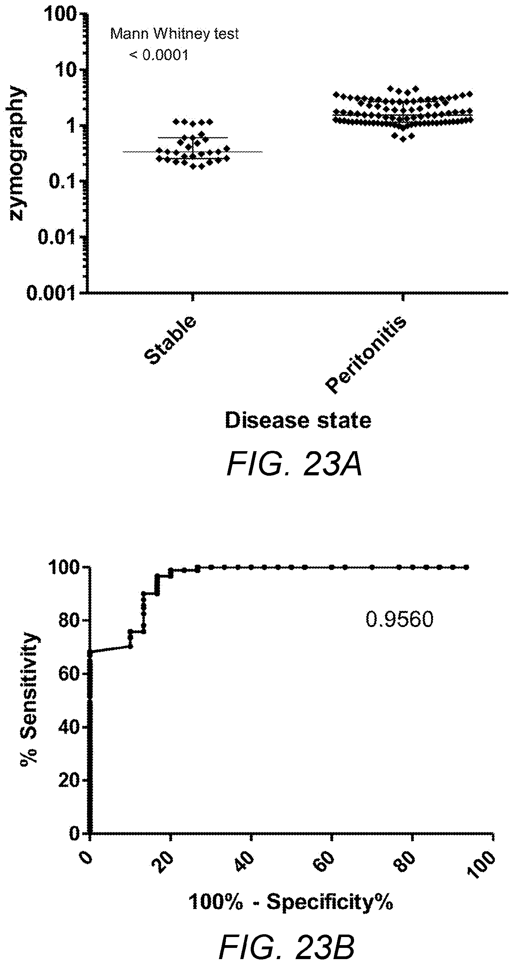

Figure 23A: Scatter graph showing median and IQR generated from zymography data comparing peritonitis vs stable individuals (Mann-Whitney test p<0.0001);
Figure 23B: Receiver Operator Characteristic (ROC) curves for the diagnosis of infection, area under the curve (AUC) values are shown;

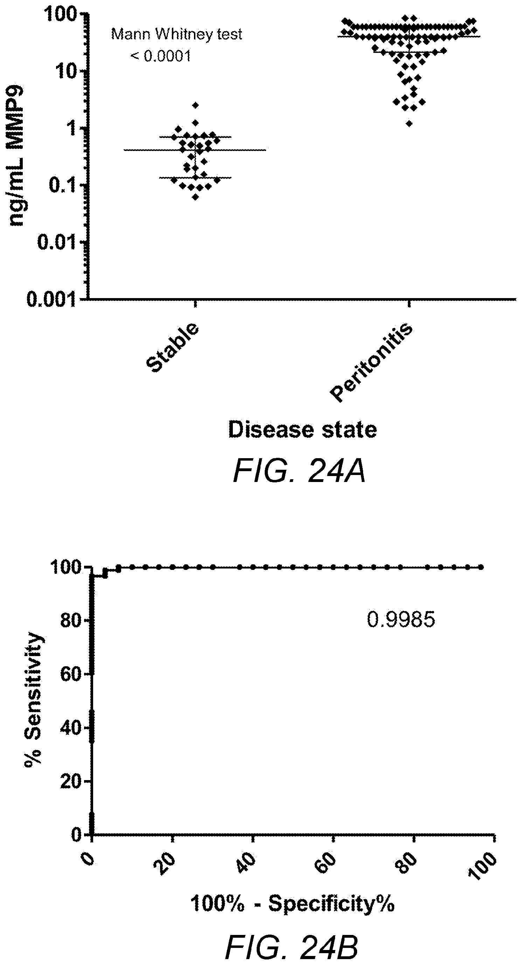

Figure 24A: Scatter graph showing median and IQR generated from MMP9 data comparing peritonitis vs stable individuals (Mann-Whitney test p<0.0001);
Figure 24B: Receiver Operator Characteristic (ROC) curves for the diagnosis of infection, area under the curve (AUC) values are shown;

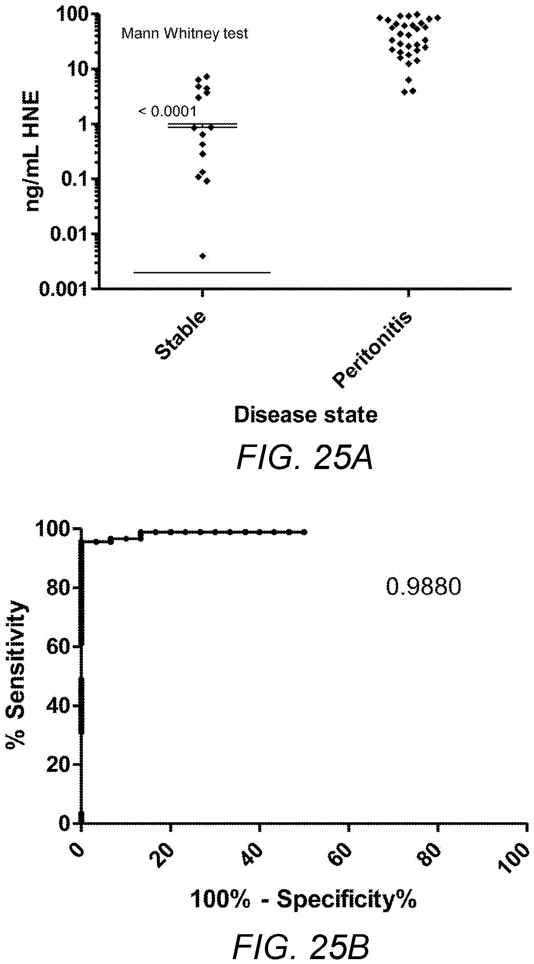

Figure 25A: Scatter graph showing median and IQR generated from HNE data comparing peritonitis vs stable individuals (Mann-Whitney test p<0.0001);
Figure 25B: Receiver Operator Characteristic (ROC) curves for the diagnosis of infection, area under the curve (AUC) values are shown;

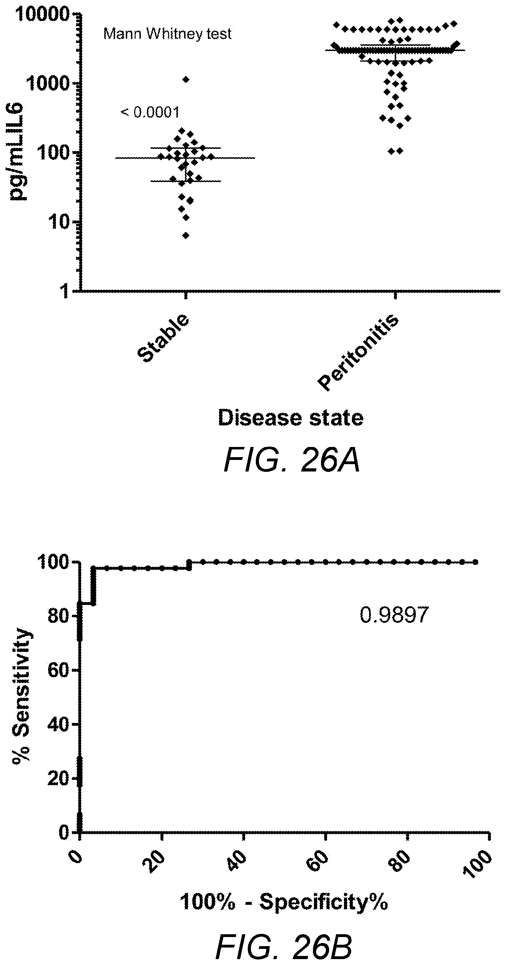

Figure 26A: Scatter graph showing median and IQR generated from IL6 data comparing peritonitis vs stable individuals (Mann-Whitney test p<0.0001);
Figure 26B: Receiver Operator Characteristic (ROC) curves for the diagnosis of infection, area under the curve (AUC) values are shown;

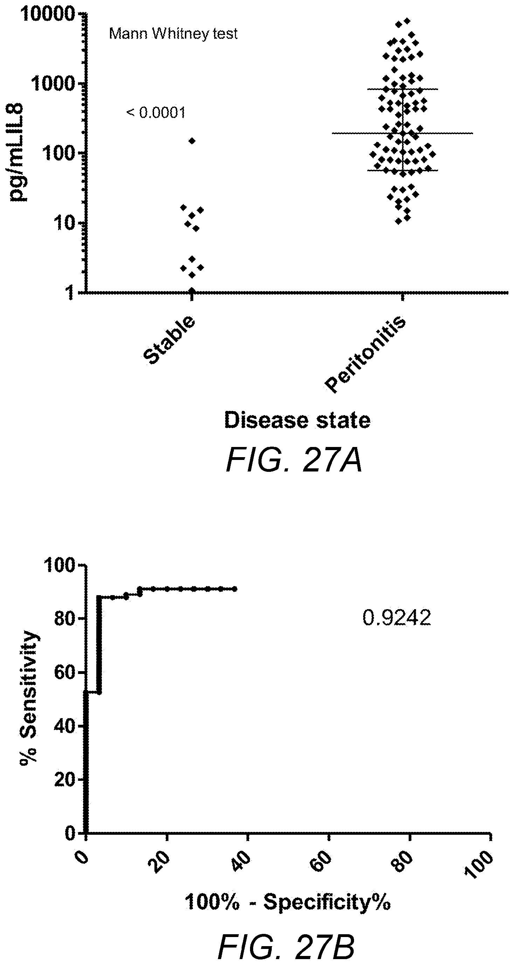

Figure 27A: Scatter graph showing median and IQR generated from IL8 data comparing peritonitis vs stable individuals (Mann-Whitney test p<0.0001);
Figure 27B: Receiver Operator Characteristic (ROC) curves for the diagnosis of infection, area under the curve (AUC) values are shown;

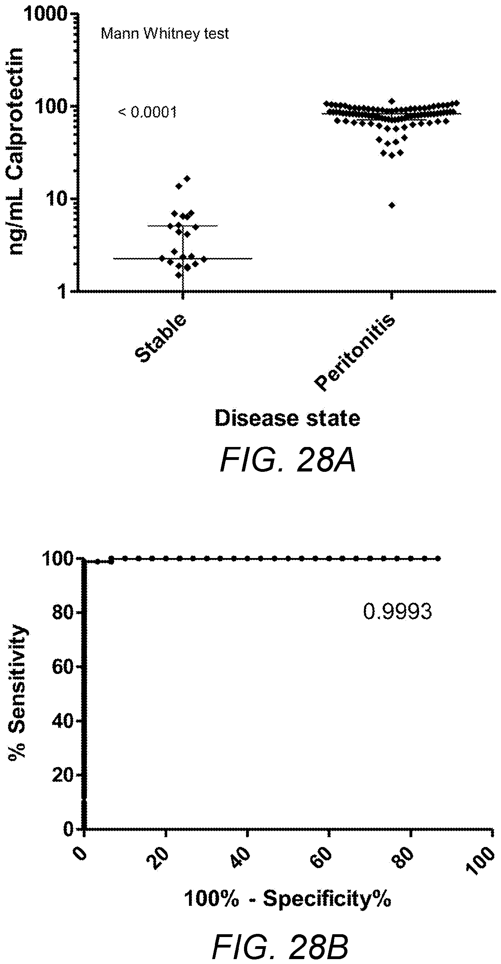

Figure 28A: Scatter graph showing median and IQR generated from calprotectin data comparing peritonitis vs stable individuals (Mann-Whitney test p<0.0001);
Figure 28B: Receiver Operator Characteristic (ROC) curves for the diagnosis of infection, area under the curve (AUC) values are shown;

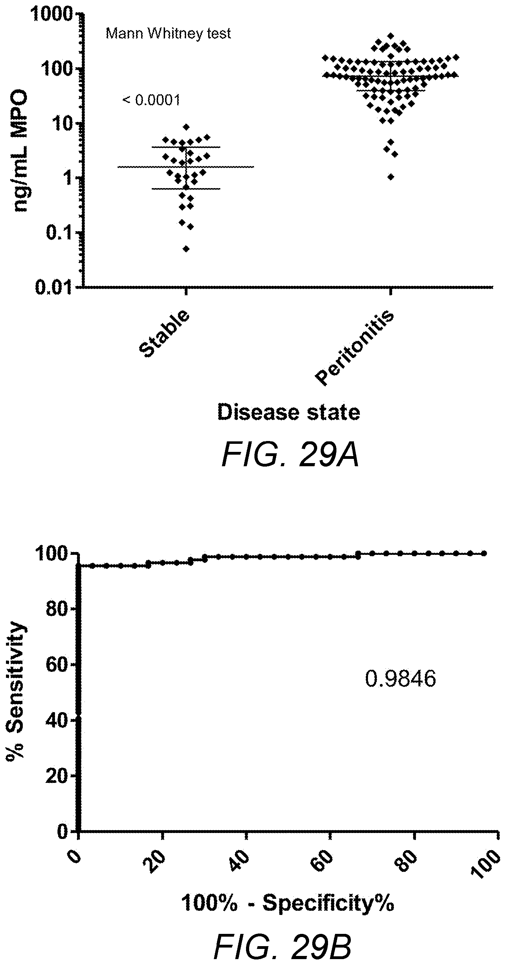

Figure 29A: Scatter graph showing median and IQR generated from MPO data comparing peritonitis vs stable individuals (Mann-Whitney test p<0.0001); and
Figure 29B: Receiver Operator Characteristic (ROC) curves for the diagnosis of infection, area under the curve (AUC) values are shown.

### SUMMARY OF THE DISCLOSURE

Disclosed is a diagnostic test apparatus comprising:
an elongate housing defining a test strip holder containing a lateral flow test strip;
a fluid sampling chamber provided with an opening connecting the fluid sampling chamber with the test strip holder;
a viewing window in the elongate housing allowing reading of one or more portions of the lateral flow test strip; and
a connector configured to couple the diagnostic test apparatus to a bulk source of fluid.

The diagnostic test apparatus advantageously allows for the testing of fluid directly as it is fed into, drained from or contained in a bulk source of fluid such as a dialysate fluid bag. This allows the patient to carry out the test without unhygienic exposure to the waste fluid. In addition, the test may be carried out while emptying the dialysate fluid bag to waste via a drain tube.

The diagnostic test apparatus may also retain a fluid sample from the bulk source of fluid during the test. This may allow the fluid sample to be conveniently retained for later testing, re-testing or analysis of the fluid sample at a remote site if needed. This is particularly advantageous where the test is carried out during draining to waste since otherwise no fluid would be obtainable for re-testing.

The connector may be configured to establish fluid communication between the bulk source of fluid and the fluid sampling chamber.

The connector may comprise an interference fit connector, an adhesive connector, or a clip connector.

The connector may comprise a tubular element. The tubular element may contain the fluid sampling chamber. The tubular element may comprise a first end and a second end and the fluid sampling chamber may be interposed between the first end and the second end.

In one example both the first end and the second end are open to thereby enable a flowing stream of fluid to pass through the fluid sampling chamber during testing. This is particularly beneficial for testing while draining a fluid to waste along a tube or while filling a container along a tube.

In another example the first end is open and the second end is closed to thereby enable a volume of fluid to pass into, and be retained within, the fluid sampling chamber during testing. This is particularly beneficial for testing a source of fluid retained in a closed container where it is desirable to prevent leakage of fluid.

The tubular element may be configured for push-fit coupling to a tube of the bulk source of fluid. The tubular element may comprise an O-ring seal for engaging the tube of the bulk source of fluid.

Alternatively, the connector may comprise a clip for clipping to a tube of the bulk source of fluid.

Alternatively, the tubular element may comprise an adhesive element for adhering to a surface of a container of the bulk source of fluid. The adhesive element may comprise an adhesive pad provided on an end of the tubular element.

The connector may be integral with the elongate housing.

The diagnostic test apparatus may further comprise a puncturing element for opening fluid communication between the bulk source of fluid and the fluid sampling chamber.

The puncturing element may be contained within the fluid sampling chamber.

The puncturing element may puncture an element of the bulk source of fluid during coupling of the connector to the bulk source of fluid. Alternatively, the puncturing element may puncture an element of the bulk source of fluid after coupling of the connector to the bulk source of fluid.

The puncturing element may comprises a piercing element. The piercing element may be statically-mounted within the connector or movably-mounted within the connector to be movable from a retracted position to an extended position. The piercing element may be manually movable into the extended position by operation of a twistable and/or pushable plunger. The puncturing element may comprise a pin, tube or blade.

The diagnostic test apparatus may further comprise a wick element for wicking fluid from the fluid sampling chamber into the test strip holder.

The wick element may project through the opening into the fluid sampling chamber.

The wick element may comprise an end portion of the lateral flow test strip. Alternatively, the wick element may comprise a separate wick element that is in fluid contact with the lateral flow test strip.

The wick element comprises an elongate wick or a tubular or part-tubular element.

The wick may comprises a foam element.

The fluid sampling chamber may be located at a first end of the elongate housing.

The fluid sampling chamber may be formed integrally with the elongate housing.

In a further aspect of the present disclosure there is provided a kit of parts comprising:
a diagnostic test apparatus as described above; and
a bulk source of fluid.

The bulk source of fluid may comprise a container holding the fluid and the connector of the diagnostic test apparatus may be configured to be coupled to the container.

The container may be closed prior to coupling of the diagnostic test apparatus.

The container may comprise a storage bag and one or more tubes extending from the storage bag.

The connector of the diagnostic test apparatus may be configured to be coupled to the storage bag, for example to be coupled to the one or more tubes.

The container may hold greater than 100ml, optionally greater than 250ml, optionally greater than 1000ml of fluid.

The container may be a dialysate bag, optionally a peritoneal dialysate bag.

The fluid may be wicked from the fluid sampling chamber through the opening.

The connector may be interference fit, adhered, or clipped to the bulk source of fluid.

Fluid may be conveyed from the bulk source of fluid through the fluid sampling chamber as a flowing stream of fluid that passes through the fluid sampling chamber during testing. Alternatively, fluid may be conveyed from the bulk source of fluid into, and retained within, the fluid sampling chamber during testing.

The fluid communication between the bulk source of fluid and the fluid sampling chamber of the diagnostic test apparatus may be established by puncturing an element of the bulk source of fluid.

The fluid communication between the bulk source of fluid and the fluid sampling chamber of the diagnostic test apparatus may be maintained for a period of time to sufficiently saturate the test strip as would be readily understood by one skilled in the art. This may be a period of around 1-10 seconds, such as around 5 seconds. An indicator of wetting of the test strip may be included to confirm to the user that sufficient sample has reached the viewing window. This may be a colour change on the test strip for example. Once this indicator has been detected, the diagnostic test apparatus may then be disconnected from the bulk source of fluid.

The bulk source of fluid may be punctured during coupling of the connector to the bulk source of fluid. Alternatively, the bulk source of fluid may be punctured after coupling of the connector to the bulk source of fluid.

The element of the bulk source of fluid may be punctured by driving the element against a puncturing element of the connector. Alternatively, the element of the bulk source of fluid may be punctured by driving a puncturing element of the connector through the element of the bulk source of fluid.

The bulk source of fluid may comprise a container holding the fluid and the connector of the diagnostic test apparatus may be configured to be coupled to the container.

The container may be closed prior to coupling of the diagnostic test apparatus.

The container may be a dialysate bag, optionally a peritoneal dialysate bag.

The bulk source of fluid may comprise a continuous flow of fluid along a drain line and the connector of the diagnostic test apparatus may be configured to be coupled to the drain line.

### Detailed Description

In the following the present disclosure will be described by way of example only for the testing of dialysate fluid for peritonitis infection where the dialysate fluid is contained in or flowing into a dialysate bag. It will be readily appreciated that the diagnostic test apparatus, kit of parts and methods herein may be utilised with other fluids and other containers and for testing for different substances and conditions.

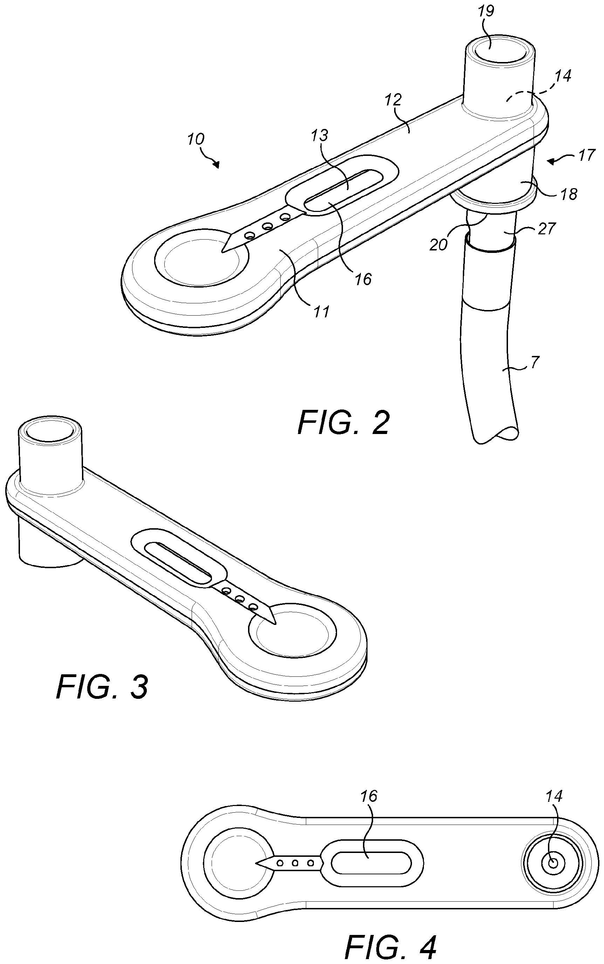

A first example of a diagnostic test apparatus 10 according to the present disclosure is shown in Figures 2 to 5 and comprises an elongate housing 11 defining a test strip holder 12 which contains a lateral flow test strip 13. A fluid sampling chamber 14 is provided that connects with the test strip holder 12 via an internal fluid opening. The elongate housing 11 is provided with a viewing window 16 allowing viewing and reading of a portion of the lateral flow test strip 13.

The lateral flow test strip 13 may project into the fluid sampling chamber 14 through the opening. Alternatively, a wick element may be provided for wicking fluid from the fluid sampling chamber through the opening into contact with the lateral flow test strip 13.

A connector 17 is provided for coupling the diagnostic test apparatus 10 to a bulk source of fluid. The connector 17 may be located at one end of the elongate housing 11 and may be formed integrally, or partly integrally therewith.

The connector 17 may comprise a tubular element 18 having an open first end 19 and an open second end 20. The fluid sampling chamber 14 may be located interposed between the first end 19 and the second end 20. As shown, the second end 20 may be coupled to a drain line 7 leading to a dialysate fluid bag. The first end 19 may be coupled to another drain line (not shown) for feeding fluid from a patient's abdomen via a catheter.

The connector 17 may comprise more than one piece. As shown, either or both of the first end 19 and second end 20 may comprise a separate tubular interconnector 27 allowing an interference push-fit coupling of the tubular element 18 with the drain lines. Alternatively, the size and shape of the first end 19 and second end 20 may be configured to directly receive the drain lines as an interference push-fit.

In use, fluid to be tested - for example fluid received from a drain line connected to a catheter - passes through the diagnostic test apparatus 10 as a stream of fluid passing from a first drain line fluidly coupled to the first end 19 of the connector 17 via the fluid sampling chamber 14 and out of the second end 20 of the connector 17 into the fluidly coupled drain line 7. The fluid flowing through the fluid sampling chamber wets the lateral flow test strip 13 either directly or via wetting of the intermediate wick element.

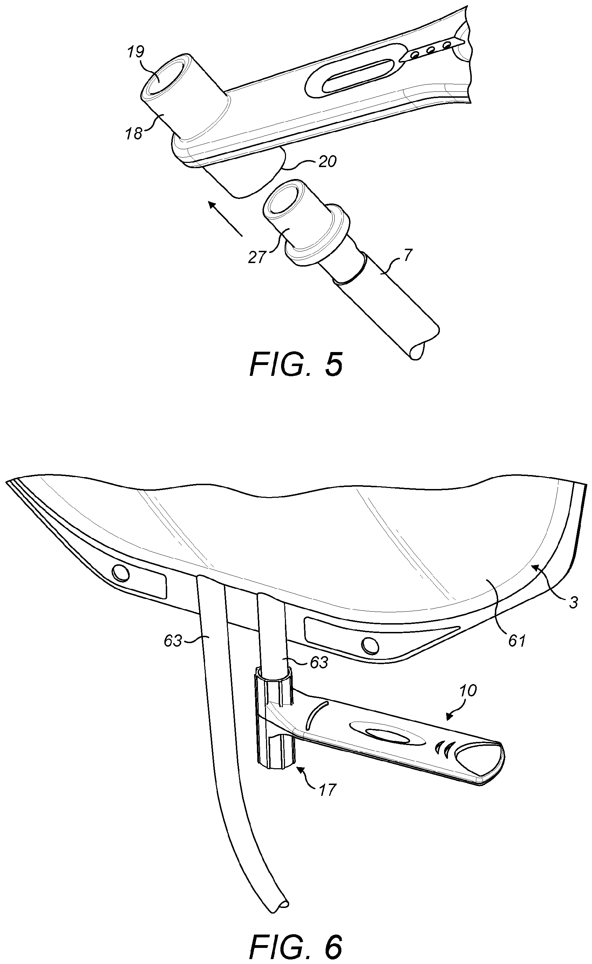

A second example of a diagnostic test apparatus 10 according to the present disclosure is shown in Figures 6 to 9. Like reference numerals have been used for like components between examples. As with the first example, the diagnostic test apparatus 10 comprises an elongate housing 11 defining a test strip holder 12 which contains the lateral flow test strip 13 and is provided with a viewing window 16 allowing viewing and reading of a portion of the lateral flow test strip 13.

As above, a connector 17 is provided for coupling the diagnostic test apparatus 10 to a bulk source of fluid. In this example the bulk source of fluid is shown as a dialysate fluid bag 3. The dialysate fluid bag 3 may comprise a flexible sac 61 having one or more tubes 63 extending therefrom. The connector 17 may be configured for coupling to an end of one of the tubes 63.

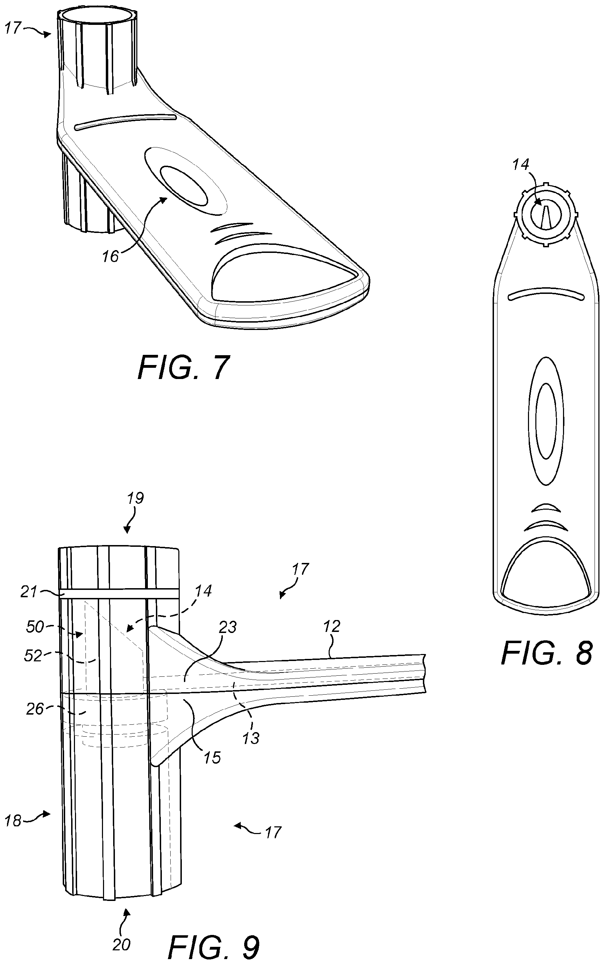

The connector 17 may be located at one end of the elongate housing 11 and may be formed integrally, or partly integrally therewith. The connector 17 may comprise a tubular element 18 as above having an open first end 19. However, in this example the second end 20 of the tubular element 18 is closed. As most clearly seen in Figure 9, the fluid sampling chamber 14 is interposed between the open first end 19 and the closed second end 20. Figure 9 also illustrates the opening 15 which may be internal and which connects the fluid sampling chamber 14 with the test strip holder 12. The lateral flow test strip 13 may project into the fluid sampling chamber 14 through the opening 15. Alternatively, a wick element 23 may be provided for wicking fluid from the fluid sampling chamber 14 through the opening 15 into contact with the lateral flow test strip 13.

The connector 17 also contains a puncturing element 50 for establishing fluid communication with the dialysate fluid bag 3. In the example shown the puncturing element 50 comprises a piercing tube 52, which may be hollow and may be statically-mounted within the fluid sampling chamber 14. An O-ring seal 21 may be provided at the open first end 19.

The fluid sampling chamber 14 may contain a foam element 26. The foam element 26 may be a foam ring that surrounds the piercing tube 52. The foam element 26 may assist in transferring fluid from the fluid sampling chamber 14 to the wick element 23. Alternatively, the foam element 26 may act itself as the wick element and be in direct contact with the lateral flow test strip 13.

As shown, the first end 19 may be coupled to an end of a tube 63 of the dialysate fluid bag 3. Typically the tube 63 will be initially closed off to prevent leakage of fluid by means of a bung. In order to couple the connector 17 to the dialysate fluid bag 3, the end of the tube 63 is pushed into the open first 19 of the connector 17 causing puncturing of the bung by the piercing tube 52. In this way fluid communication between the dialysate fluid bag 3 and the fluid sampling chamber 14 is established allowing fluid to flow into the fluid sampling chamber 14 to wet the lateral flow test strip 13 either directly or via the wick element 23 and/or the foam element 26. The closed second end 20 of the tubular element 18 and the engagement of the O-ring seal 21 on the tube 63 prevents leakage of fluid outside the diagnostic test apparatus 10.

The foam element 26 may also absorb and retain a fluid sample within the diagnostic test apparatus 10. This may allow the diagnostic test apparatus 10 to be retained for later testing, re-testing or analysis of the fluid sample at a remote site. Suitable means may be provided, for example a push-fit cap, for closing off the open first end 19 of the tubular element 18 after the initial test to assist in retaining the fluid sample.

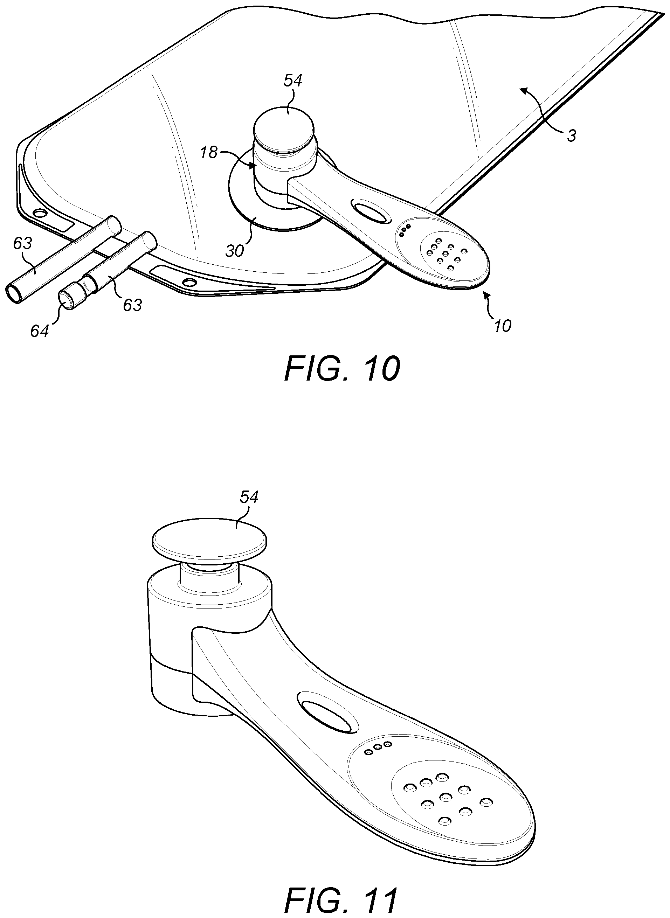

A third example of a diagnostic test apparatus 10 according to the present disclosure is shown in Figures 10 to 13. Like reference numerals have been used for like components between examples. As with the previous examples, the diagnostic test apparatus 10 comprises an elongate housing 11 defining a test strip holder 12 which contains the lateral flow test strip 13 and is provided with a viewing window 16 allowing viewing and reading of a portion of the lateral flow test strip 13.

As above, a connector 17 is provided for coupling the diagnostic test apparatus 10 to a bulk source of fluid. In this example the bulk source of fluid is again shown as a dialysate fluid bag 3 having a flexible sac 61 with one or more tubes 63 extending therefrom. The tubes 63 may be closed off using bungs 64.

In this example the connector 17 may be configured for coupling directly to the flexible sac 61, for example to a surface of the flexible sac 61.

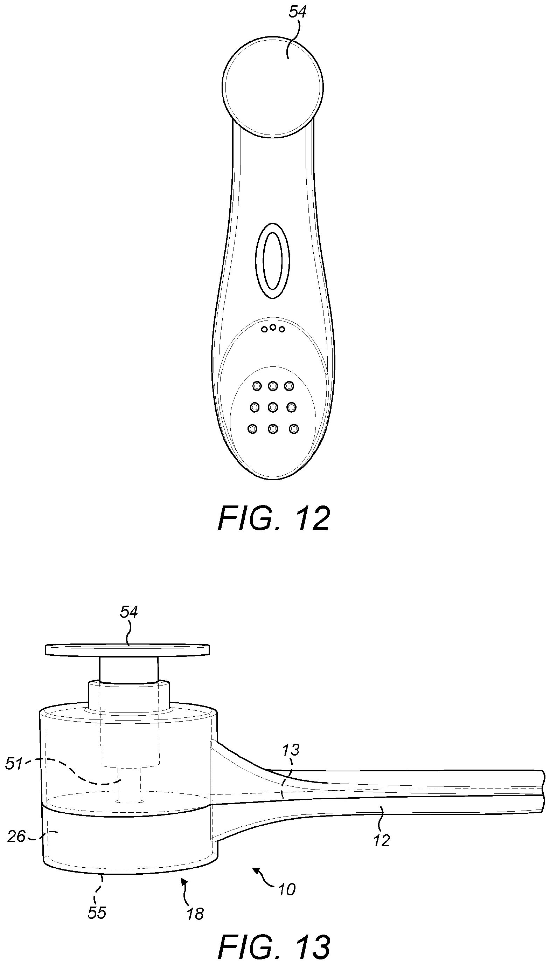

The connector 17 may be located at one end of the elongate housing 11 and may be formed integrally, or partly integrally therewith. The connector 17 may comprise a tubular element 18 defining a fluid sampling chamber 14 therein. The connector 17 also contains a puncturing element 50 for establishing fluid communication with the dialysate fluid bag 3. In the example shown the puncturing element 50 comprises a hollow piercing pin 51 within the fluid sampling chamber 14. The hollow piercing pin 51 may be movably-mounted to be movable between a retracted position, as shown in Figure 13, wherein the hollow piercing pin 51 is fully within the fluid sampling chamber and an extended position in which the hollow piercing pin 51 is driven outwards through an aperture provided in the otherwise closed second end 20 of the tubular element 18. The first end 19 of the tubular element 18 is provided with a plunger 54 which is operatively connected to the hollow piercing pin 51. The plunger 54 may be operated by pushing or twisting the plunger 54.

As above, the fluid sampling chamber 14 is interposed between the first end 19 and the second end 20 of the tubular element 18. As above, the lateral flow test strip 13 may project into the fluid sampling chamber 14 through the opening 15 or alternatively, a wick element 23 may be provided for wicking fluid from the fluid sampling chamber 14 through the opening 15 into contact with the lateral flow test strip 13.

The fluid sampling chamber 14 may contain a foam element 26. The foam element 26 may be a foam ring that surrounds the hollow piercing pin 51. The foam element 26 may assist in transferring fluid from the fluid sampling chamber 14 to the wick element 23. Alternatively, the foam element 26 may act itself as the wick element and be in direct contact with the lateral flow test strip 13.

As shown in Figure 10, the second end 20 may be provided with an adhesive pad 30 for adhering the connector 17 to a wall of the flexible sac 61. A further seal element, for example an O-ring seal, may be provided on an underside of the adhesive pad 30.

After coupling of the connector 17 to the flexible sac 61, fluid communication between the dialysate fluid bag 3 and the fluid sampling chamber 14 may be established by operating the plunger 54 to drive the hollow piercing pin 51 into the extended position in which it is driven through and punctures the wall of the flexible sac 61 allowing fluid to flow into the fluid sampling chamber 14 via the foam element 26 and/or the wick element 23.

The foam element 26 may also absorb and retain a fluid sample within the diagnostic test apparatus 10. This may allow the diagnostic test apparatus 10 to be retained for later testing, re-testing or analysis of the fluid sample at a remote site. Suitable means may be provided, for example an adhesive seal, for closing off the aperture in the second end 20 of the tubular element 18 after the initial test to assist in retaining the fluid sample.

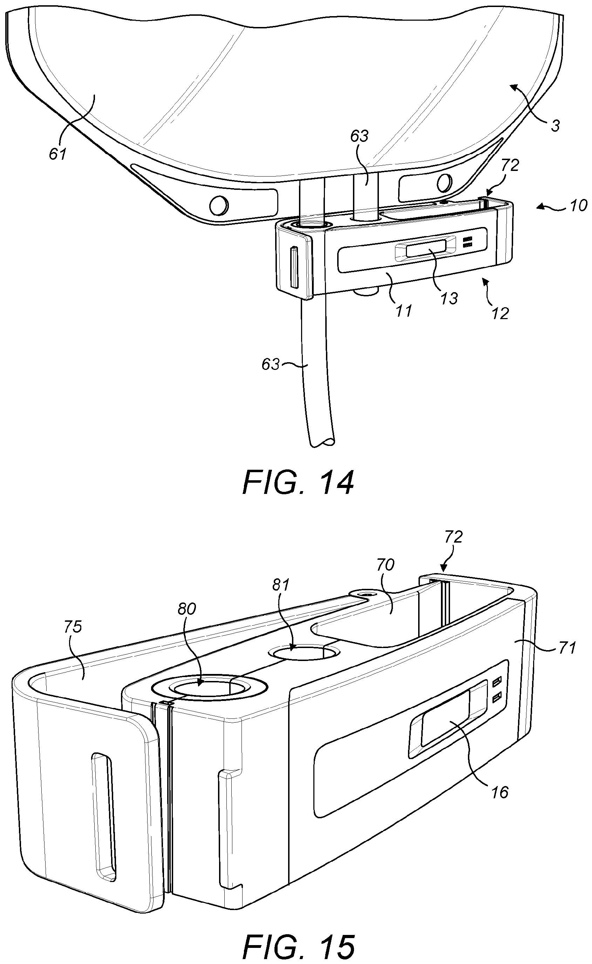

A fourth example t of a diagnostic test apparatus 10 according to the present disclosure is shown in Figures 14 to 19. Like reference numerals have been used for like components between examples. As with the previous examples, the diagnostic test apparatus 10 comprises an elongate housing 11 defining a test strip holder 12 which contains the lateral flow test strip 13 and is provided with a viewing window 16 allowing viewing and reading of a portion of the lateral flow test strip 13.

As above, a connector 17 is provided for coupling the diagnostic test apparatus 10 to a bulk source of fluid. In this example the bulk source of fluid is again shown in Figure 14 as a dialysate fluid bag 3 having a flexible sac 61 with one or more tubes 63 extending therefrom. The tubes 63 may be closed off using bungs 64.

In this example the connector 17 may be configured for coupling directly to one or both tubes 63 with a clipping action.

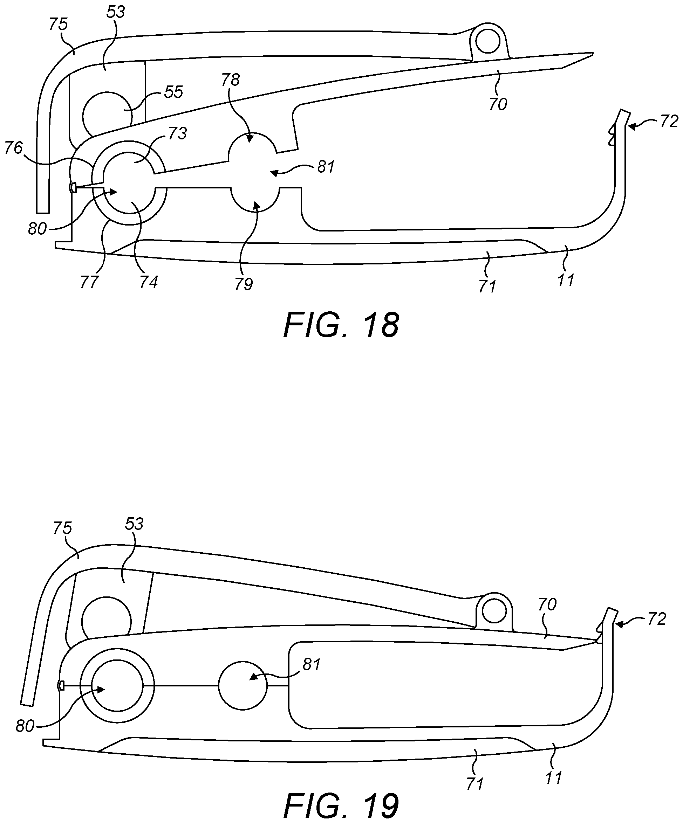

The diagnostic test apparatus 10 may comprise two clamping legs 70, 71 wherein one of the clamping legs may comprise the elongate housing 11. The two clamping legs 70, 71 may be moved between an open configuration as shown in Figure 18 and a closed configuration as shown in Figure 19. A retention clip 72 may be provided at the distal ends of the clamping legs 70, 71 which allows the diagnostic test apparatus 10 to be at least temporarily retained in the closed configuration without manual intervention.

The connector 17 may comprise a tubular element 18 formed by the interaction of the two clamping legs 70, 71. As shown in Figure 15 and 18, each clamping leg may be provided with a part-tubular recess 73, 74, optionally semi-tubular recesses, that when brought together in the closed configuration define a tubular cavity 80 therebetween. The tubular cavity 80 may be provided with a foam element 26 which may be in the form of two part-tubular foam pieces 76, 77 lining the two part-tubular recesses 73, 74. In the closed configuration the two part-tubular foam pieces 76, 77 may be brought into contact and alignment to provide a tubular foam element that lines the tubular cavity 80.

Optionally, each clamping leg 70, 71 may further be provided with an additional part-tubular recess 78, 79, optionally semi-tubular recesses, that when brought together in the closed configuration define an additional tubular cavity 81 therebetween.

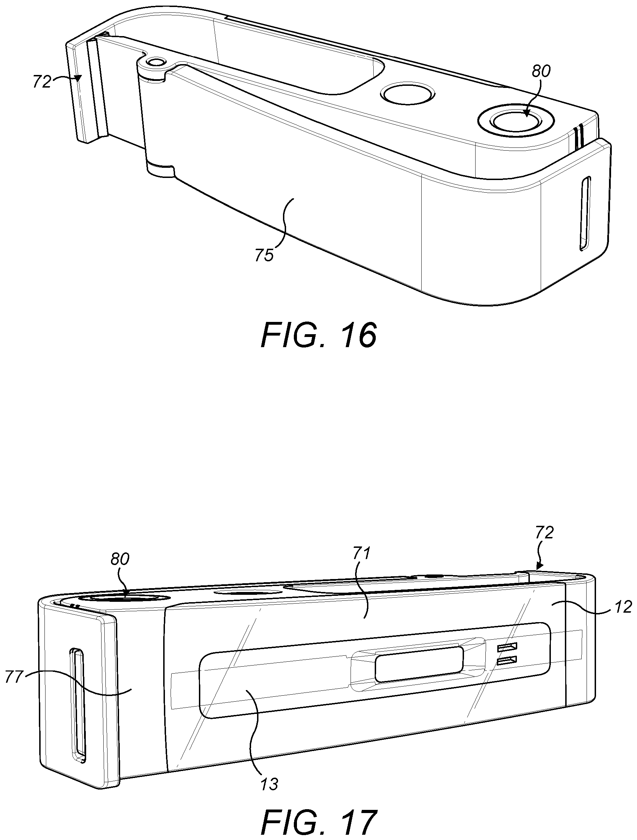

The elongate housing 11 as shown in Figure 17 comprises a fluid sampling chamber 14 that communicates with or is formed by the part-tubular foam piece 77 in the tubular cavity 80 and also the test strip holder 12 via an opening 15. As above, the lateral flow test strip 13 may extend directly through the opening 15 into contact with the foam element 26 in the form of the tubular foam element or a wick element 23 may be interposed.

The connector 17 also contains a puncturing element 50 for establishing fluid communication with the dialysate fluid bag 3. In the example shown the puncturing element 50 comprises a blade 53 which is movable into the tubular cavity 80 when the clamping legs 70, 71 are in the closed configuration. As shown in Figure 18, the blade 53 may be mounted on a pivotable third leg 75 which may be pivotally connected to one of the clamping legs 70, 71. The blade 53 may be pivoted into the tubular cavity 80 of the tubular element 18 though an aperture provided in the one of the clamping legs 70, 71.

The diagnostic test apparatus 10 may be coupled to one or two tubes 63 of the dialysate fluid bag 3 as shown in Figure 14 by clipping the tubes 63 into the tubular cavity 80 and additional tubular cavity 81.

After or during coupling of the connector 17 to the one or more tubes 63, fluid communication between the dialysate fluid bag 3 and the fluid sampling chamber 14 may be established by pivoting the third leg 75 to drive the blade 53 into the tubular cavity 80 wherein it is driven through and punctures the wall of the tube 63 allowing fluid to flow into the tubular cavity 80 where it soaks the tubular foam element. Fluid is then transferred via the tubular foam element onto the lateral flow test strip 13.

The blade 53 may be provided with a central aperture 55 which allows fluid to pass across the plane of the blade 53. This may allow, after puncturing of the tube 63, fluid to be drained from the dialysate fluid bag 3 why carrying out the test. In other words fluid may freely flow through the tubular cavity 80 to drain while wetting the foam element sufficiently to transfer fluid to the lateral flow test strip 13.

The foam element 26 may also absorb and retain a fluid sample within the diagnostic test apparatus 10. This may allow the diagnostic test apparatus 10 to be retained for later testing, re-testing or analysis of the fluid sample at a remote site. Suitable means may be provided, for example adhesive seals or caps, for closing off the tubular cavity 80 after the initial test to assist in retaining the fluid sample.

### Example 1 - Pilot data to show selection of biomarkers

Waste PD fluid was collected from 91 patients with peritonitis and 30 stable patients and stored at -80°C. All samples were analysed for more than 50 potential biomarkers using a variety of reference assays (immunoassays, zymography and protease substrate assays).

Of the potential biomarkers analysed MMP8 was found to be significantly elevated in peritonitis patients compared to stable PD patients.

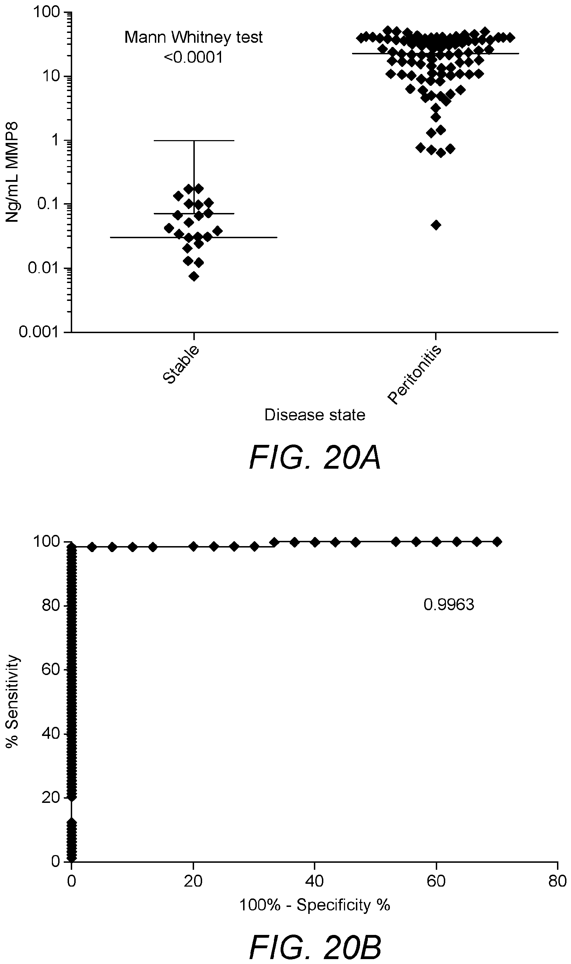

The median level of MMP8 levels in waste PD fluid was 0.032ng/mL (IQR=0.0ng/mL - 0.076ng/mL) compared to 23.09ng/mL (IQR=10.62ng/mL - 37.91ng/mL) in individuals suffering from peritonitis (Figure 20A). This marker has excellent diagnostic accuracy with a ROC AUC of 0.9963 (positive predictive value = 1.00, negative predictive value = 0.97; figure 20B and table 2).

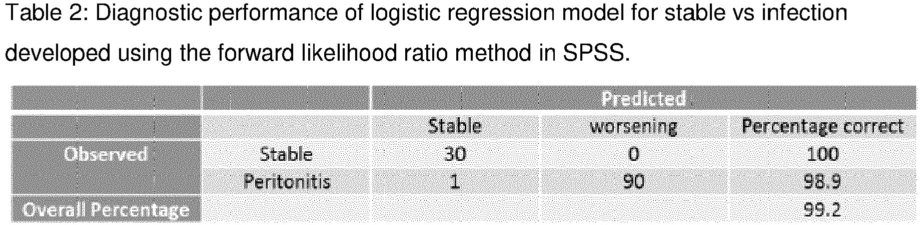

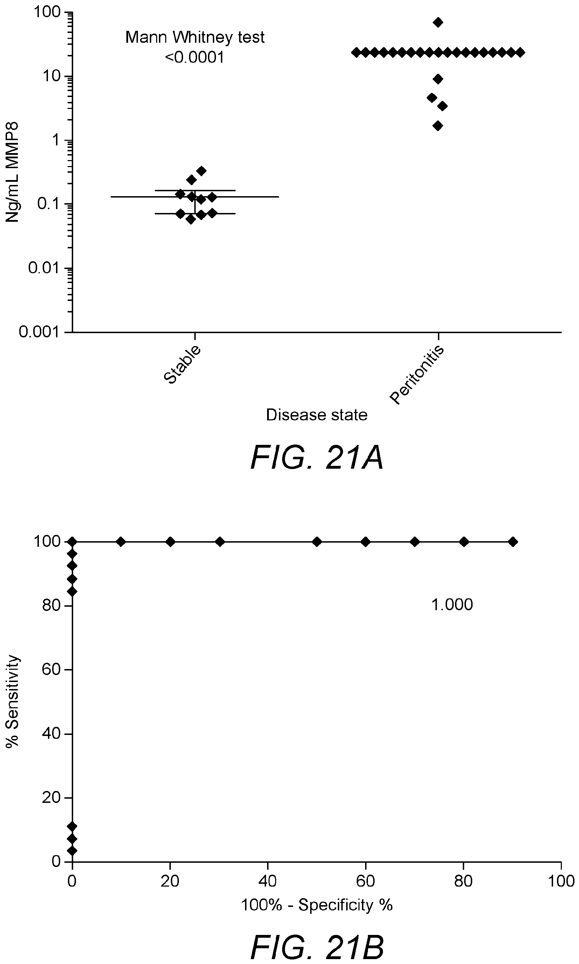

The ability of the assay to perform in a lateral flow format was investigated. For a subset of the samples (peritonitis n=26 and stable n=10) the LF devices produced equivalent results to the ELISA (Spearman's rank = 0.808, p <0.001) with an ROC AUC of 1 and a p value of <0.001. Results are shown in Figure 21A and 21B.

### MMP8 Decision Tree to determine cut-off values:

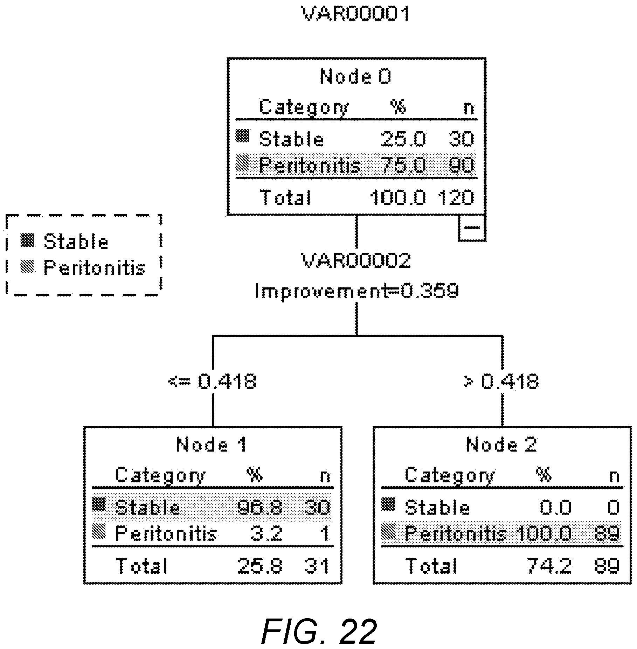

The preliminary study provided a cut-off value of 0.418ng/mL using decision tree analysis (SPSS). Results are shown in Figure 22 and performance is summarised in Table 3 below:

**Classification**

| Observed | Predicted | | |
|---|---|---|---|
| | Stable | Peritonitis | Percent Correct |
| Stable | 30 | 0 | 100.0% |
| Peritonitis | 1 | 89 | 98.9% |
| Overall Percentage | 25.8% | 74.2% | 99.2% |

| | | | |
|---|---|---|---|
| Growing Method: CRT Dependent Variable: VAR00001 | | | |

The performance of a selection of the biomarkers tested is summarised in Table 4 below:

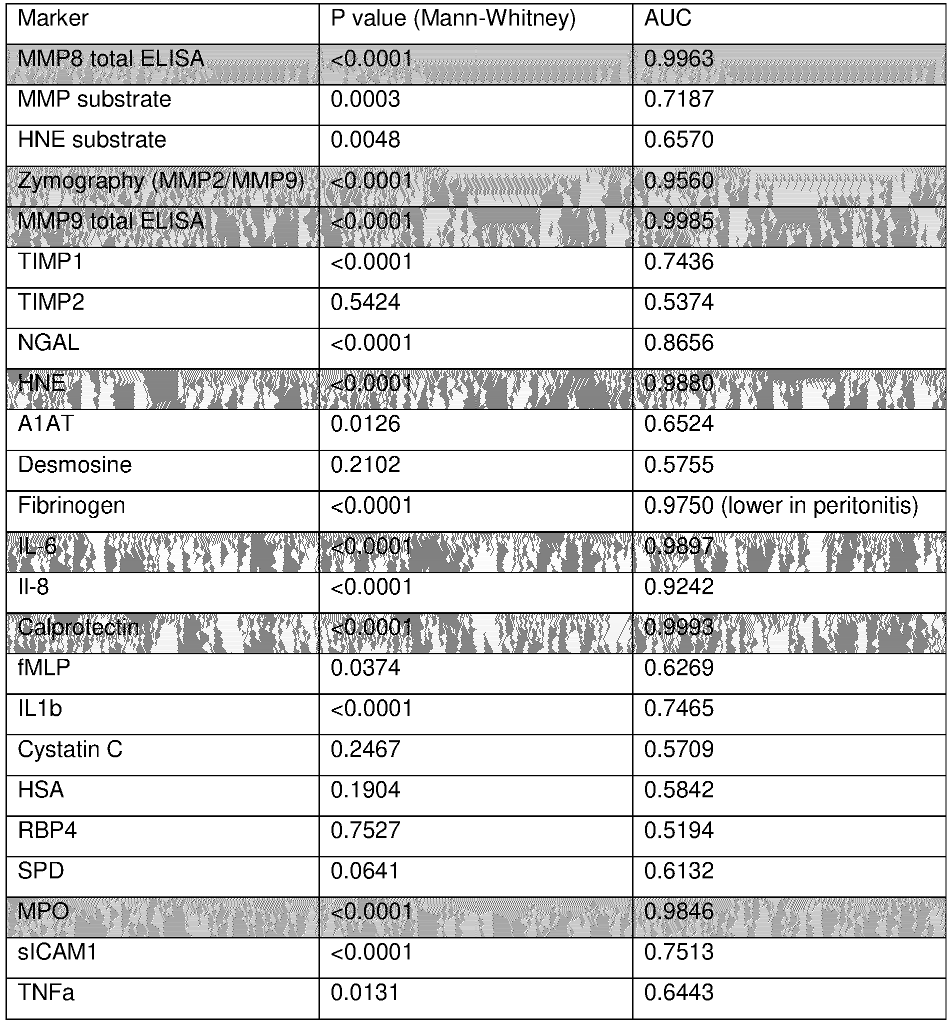

## Claims

1. A method of detecting peritonitis caused by an infection in a subject suffering from kidney disease and undergoing peritoneal dialysis, the method comprising determining the level of at least the marker MMP8 in a sample of peritoneal dialysate wherein an increased level of at least MMP8 is indicative of peritonitis caused by an infection.

2. The method according to claim 1, further comprising selecting the subject for treatment with an antibiotic based on an increased level of at least MMP8 in the sample of peritoneal dialysate.

3. The method according to claim 1 or 2, comprising determining the level of at least two markers in a sample of peritoneal dialysate, wherein one of the markers is MMP8 and the other marker is selected from IL-6, HNE, MMP2, MMP9, TIMP1, TIMP2, NGAL, A1AT, desmosine, fibrinogen, IL-8, calprotectin, fMLP, IL1b, cystatin C, HSA, RBP4, SPD, MPO, sICAM and TNFa, wherein an increased level of MMP8 and at least one of IL-6, HNE, MMP2, MMP9, TIMP1, TIMP2, NGAL, A1AT, desmosine, IL-8, calprotectin, fMLP, IL1b, cystatin C, HSA, RBP4, SPD, MPO, sICAM and TNFa and/or a decreased level of fibrinogen is indicative of peritonitis caused by an infection.

4. The method of claims 3, wherein the at least one marker is selected from MMP8 and the other marker is selected from HNE, MMP2, MMP9, IL-6, calprotectin and MPO.

5. The method according to claim 3 or 4, wherein the level of the markers MMP8 and IL-6 is determined in a sample of peritoneal dialysate.

6. The method according to any one of claims 1-5, wherein the method comprises detecting peritonitis at an early stage.

7. The method according to any one of claims 1-6, wherein the method is performed in a lateral flow assay format.

8. The method of claim 7, which is performed on a bulk source of fluid using a diagnostic test apparatus of the type comprising:
an elongate housing defining a test strip holder containing a lateral flow test strip, a fluid sampling chamber provided with an opening connecting the fluid sampling chamber with the test strip holder, and a viewing window in the elongate housing allowing reading of one or more portions of the lateral flow test strip;
the method comprising the steps of:
coupling the diagnostic test apparatus to the bulk source of fluid by use of a connector;
during or after coupling of the connector, establishing a fluid communication between the bulk source of fluid and the fluid sampling chamber of the diagnostic test apparatus to convey fluid from the bulk source of fluid into the fluid sampling chamber;
causing fluid to pass from the fluid sampling chamber through the opening to wet the lateral flow test strip.

9. The method of claim 7, which is performed using a diagnostic test apparatus comprising:
an elongate housing defining a test strip holder containing a lateral flow test strip;
a fluid sampling chamber provided with an opening connecting the fluid sampling chamber with the test strip holder;
a viewing window in the elongate housing allowing reading of one or more portions of the lateral flow test strip; and
a connector configured to couple the diagnostic test apparatus to a bulk source of fluid.

10. An antibiotic for use in a method of treating peritonitis, wherein the subject suffers from kidney disease and is undergoing peritoneal dialysis and has been selected for treatment by performing the method of any one of claims 1 to 9.

11. The antibiotic for use of claim 10, wherein the subject displays an increased level of at least MMP8 and IL-6.

12. The antibiotic for use of any of claims 10 to 11, wherein the antibiotic is:
a) selected from an aminoglycoside, a cephalosporin, a glycopeptide, a penicillin, a quinolone, aztreonam, clindamycin, imipenem-cilastin, linezolid, metronidazole, rifampin and an antifungal and/or
b) administered intraperitoneally, orally or intravenously (preferably intraperitoneally); and/or
c) administered during peritoneal dialysis and/or
d) administered with the dialysis fluid utilised in the peritoneal dialysis and/or
e) administered separately from the dialysis fluid utilised in the peritoneal dialysis.

## Patentansprüche

1. Verfahren zur Erkennung einer Peritonitis, die durch eine Infektion in einem an Nierenerkrankung leidenden und sich einer Peritonealdialyse unterziehenden Individuum verursacht wurde, wobei das Verfahren die Bestimmung des Spiegels mindestens des Markers MMP8 in einer Probe von Peritonealdialysat umfasst, wobei ein erhöhter Spiegel von mindestens MMP8 auf eine durch eine Infektion verursachte Peritonitis hinweist.

2. Verfahren nach Anspruch 1 ferner umfassend das Auswählen des Individuums zur Behandlung mit einem Antibiotikum auf Basis eines erhöhten Spiegels von mindestens MMP8 in der Probe des Peritonealdialysats.

3. Verfahren nach Anspruch 1 oder 2, umfassend das Bestimmen der Konzentration von mindestens zwei Markern in einer Probe von Peritonealdialysat, wobei einer der Marker MMP8 ist und der andere Marker ausgewählt ist aus IL-6, HNE, MMP2, MMP9, TIMP1, TIMP2, NGAL, A1AT, Desmosin, Fibrinogen, IL-8, Calprotectin, fMLP, IL1b, Cystatin C, HSA, RBP4, SPD, MPO, sICAM und TNFa, wobei ein erhöhter Spiegel von MMP8 und mindestens einem von IL-6, HNE, MMP2, MMP9, TIMP1, TIMP2, NGAL, A1AT, Desmosin, IL-8, Calprotectin, fMLP, IL1b, Cystatin C, HSA, RBP4, SPD, MPO, sICAM und TNFa und/oder ein verminderter Spiegel an Fibrinogen auf eine durch eine Infektion verursachte Peritonitis hinweist.

4. Verfahren nach Anspruch 3, wobei der mindestens eine Marker aus MMP8 ausgewählt wird und der andere Marker aus HNE, MMP2, MMP9, IL-6, Calprotectin und MPO ausgewählt wird.

5. Verfahren nach Anspruch 3 oder 4, wobei der Spiegel der Marker MMP8 und IL-6 in einer Probe von Peritonealdialysat bestimmt wird.

6. Verfahren nach einem der Ansprüche 1-5, wobei das Verfahren das Erkennen von Peritonitis in einem frühen Stadium umfasst.

7. Verfahren nach einem der Ansprüche 1-6, wobei das Verfahren in einem Lateral-Flow-Assay-Format durchgeführt wird.

8. Verfahren nach Anspruch 7, wobei das Verfahren an einer Hauptquelle von Fluid unter Verwendung einer diagnostischen Testvorrichtung des Typs durchgeführt wird, der umfasst:
ein längliches Gehäuse, das einen Teststreifenhalter definiert, der einen Lateral-Flow-Teststreifen enthält, eine Fluidproben-Entnahmekammer, die mit einer Öffnung bereitgestellt ist, die die Fluidproben-Entnahmekammer mit dem Teststreifenhalter verbindet, und ein Sichtfenster in dem länglichen Gehäuse, das das Ablesen eines oder mehrerer Abschnitte des Lateral-Flow-Teststreifens ermöglicht;
wobei das Verfahren die folgenden Schritte umfasst:
Koppeln der diagnostischen Testvorrichtung an die Fluid-Hauptquelle unter Verwendung eines Verbinders;
während oder nach dem Koppeln des Verbinders, Einrichten einer fluidleitenden Verbindung zwischen der Fluid-Hauptquelle und der Fluidproben-Entnahmekammer der diagnostischen Testvorrichtung, um Fluid von der Fluid-Hauptquelle in die Fluidproben-Entnahmekammer zu befördern;
Passierenlassen von Fluid aus der Fluidproben-Entnahmekammer durch die Öffnung, um den Lateral-Flow-Teststreifen zu benetzen.

9. Verfahren nach Anspruch 7, das unter Verwendung einer diagnostischen Testvorrichtung durchgeführt wird, die Folgendes umfasst:
ein längliches Gehäuse, das einen Teststreifenhalter definiert, der einen Lateral-Flow-Teststreifen enthält;
eine Fluidproben-Entnahmekammer, die mit einer Öffnung bereitgestellt ist, welche die Fluidproben-Entnahmekammer mit dem Teststreifenhalter verbindet;
ein Sichtfenster in dem länglichen Gehäuse, das das Ablesen von einem oder mehreren Abschnitten des Lateral-Flow-Teststreifens ermöglicht; und
einen Verbinder, der konfiguriert ist, um die diagnostische Testvorrichtung mit einer Hauptquelle von Fluid zu koppeln.

10. Antibiotikum zur Verwendung in einem Verfahren zur Behandlung von Peritonitis, wobei das Individuum an einer Nierenerkrankung leidet und sich einer Peritonealdialyse unterzieht und zur Behandlung unter Durchführung des Verfahrens nach einem der Ansprüche 1 bis 9 ausgewählt ist.

11. Antibiotikum zur Verwendung nach Anspruch 10, wobei das Individuum einen erhöhten Spiegel von mindestens MMP8 und IL-6 aufweist.

12. Antibiotikum zur Verwendung nach einem der Ansprüche 10 bis 11, wobei das Antibiotikum:
a) ausgewählt ist aus einem Aminoglykosid, einem Cephalosporin, einem Glykopeptid, einem Penicillin, einem Chinolon, Aztreonam, Clindamycin, Imipenem-Cilastin, Linezolid, Metronidazol, Rifampin und einem Fungizid und/oder
b) intraperitoneal, oral oder intravenös (vorzugsweise intraperitoneal) verabreicht wird; und/oder
c) während der Peritonealdialyse verabreicht wird und/oder
d) mit dem bei der Peritonealdialyse verwendet Dialysefluid verabreicht wird; und/oder
e) getrennt von dem bei der Peritonealdialyse verwendeten Dialysefluid verabreicht wird.

## Revendications

1. Procédé de détection d'une péritonite provoquée par une infection chez un sujet souffrant d'une maladie rénale et sous dialyse péritonéale, le procédé comprenant l'étape consistant à déterminer le niveau d'au moins un marqueur MMP8 dans un échantillon de dialysat péritonéal où un niveau accru d'au moins MMP8 indique une péritonite provoquée par une infection.

2. Procédé selon la revendication 1, comprenant en outre l'étape consistant à sélectionner le sujet à des fins de traitement par un antibiotique sur la base d'un niveau accru d'au moins MMP8 dans l'échantillon de dialysat péritonéal.

3. Procédé selon la revendication 1 ou 2, comprenant l'étape consistant à déterminer le niveau d'au moins deux marqueurs dans un échantillon de dialysat péritonéal, où l'un des marqueurs est MMP8 et l'autre marqueur est choisi entre IL-6, HNE, MMP2, MMP9, TIMP1, TIMP2, NGAL, A1AT, la desmosine, le fibrinogène, IL-8, la calprotectine, fMLP, IL1b, la cystatine C, HSA, RBP4, SPD, MPO, sICAM et TNFa, où un niveau accru de MMP8 et d'au moins un marqueur entre IL-6, HNE, MMP2, MMP9, TIMP1, TIMP2, NGAL, A1AT, la desmosine, IL-8, la calprotectine, fMLP, IL1b, la cystatine C, HSA, RBP4, SPD, MPO, sICAM et TNFa et/ou un niveau réduit de fibrinogène indiquent une péritonite provoquée par une infection.

4. Procédé de la revendication 3, dans lequel l'au moins un marqueur choisi est MMP8 et l'autre marqueur est choisi entre HNE, MMP2, MMP9, IL-6, la calprotectine et MPO.

5. Procédé selon la revendication 3 ou 4, dans lequel le niveau des marqueurs MMP8 et IL-6 est déterminé dans un échantillon de dialysat péritonéal.

6. Procédé selon l'une quelconque des revendications 1 à 5, où le procédé comprend l'étape consistant à détecter une péritonite à un stade précoce.

7. Procédé selon l'une quelconque des revendications 1 à 6, où le procédé est mis en œuvre dans un format d'analyse à écoulement latéral.

8. Procédé de la revendication 7, qui est mis en œuvre sur une source de fluide globale en utilisant un appareil de test de diagnostic du type comprenant :
un boîtier allongé définissant un support de bandelette de test contenant une bandelette de test à écoulement latéral, une chambre de prélèvement de fluide pourvue d'une ouverture reliant la chambre de prélèvement de fluide au support de bandelette de test, et une fenêtre de visualisation dans le boîtier allongé permettant la lecture d'une ou plusieurs parties de la bandelette de test à écoulement latéral ;
le procédé comprenant les étapes consistant à :
coupler l'appareil de test de diagnostic à la source de fluide globale en utilisant un connecteur ;
pendant ou après le couplage du connecteur, établir une communication fluidique entre la source de fluide globale et la chambre de prélèvement de fluide de l'appareil de test de diagnostic pour acheminer le fluide dans la chambre de prélèvement de fluide à partir de la source de fluide globale ;
amener le fluide à passer depuis la chambre de prélèvement de fluide à travers l'ouverture pour mouiller la bandelette de test à écoulement latéral.

9. Procédé de la revendication 7, qui est mis en œuvre en utilisant un appareil de test de diagnostic comprenant :
un boîtier allongé définissant un support de bandelette de test contenant une bandelette de test à écoulement latéral ;
une chambre de prélèvement de fluide pourvue d'une ouverture reliant la chambre de prélèvement de fluide au support de bandelette de test ;
une fenêtre de visualisation dans le boîtier allongé permettant la lecture d'une ou plusieurs parties de la bandelette de test à écoulement latéral ; et
un connecteur configuré pour coupler l'appareil de test de diagnostic à une source de fluide globale.

10. Antibiotique pour une utilisation dans un procédé de traitement de la péritonite, où le sujet souffre d'une maladie rénale et est sous dialyse péritonéale et a été sélectionné à des fins de traitement en mettant en œuvre le procédé de l'une quelconque des revendications 1 à 9.

11. Antibiotique pour une utilisation selon la revendication 10, où le sujet présente un niveau accru des au moins MMP8 et IL-6.

12. Antibiotique pour une utilisation selon l'une quelconque des revendications 10 et 11, où l'antibiotique est :
a) choisi entre un aminoside, une céphalosporine, un glycopeptide, une pénicilline, une quinolone, un aztréonam, une clindamycine, l'imipénem-cilastine, un linézolide, un métronidazole, une rifampicine et un antifongique et/ou
b) administré par voie intrapéritonéale, orale ou intraveineuse (de préférence intrapéritonéale) ; et/ou
c) administré pendant la dialyse péritonéale et/ou
d) administré avec le liquide de dialyse utilisé dans la dialyse péritonéale et/ou
e) administré séparément du liquide de dialyse utilisé dans la dialyse péritonéale.
